# EUROPEAN PATENT SPECIFICATION

(11) **EP 1 695 740 B1**
(45) Date of publication and mention of the grant of the patent: **09.04.2014**
(21) Application number: 06075428.0
(22) Date of filing: 24.02.2006
(51) Int. Cl.: A61N 5/10

(54) **Gynaecological instrument**
Gynäkologisches Instrument
Instrument gynécologique

(30) Priority: 28.02.2005 NL 1028427
(43) Date of publication of application: 30.08.2006
(73) Proprietor: Nucletron Operations B.V., 3905 TH Veenendaal (NL)
(72) Inventor: van 't Hooft, Eric, 2930 Brasschaat (BE)
(74) Representative: Jansen, Cornelis Marinus

(56) References cited:
- US-A- 4 434 789
- US-A- 5 653 683
- US-B1- 6 312 375
- US-B1- 6 390 968
- TYRIE L K ET AL: "Intrauterine high dose rate afterloading brachytherapy: Experience of fractionated therapy using a cervical sleeve technique" CLINICAL ONCOLOGY, W.B. SAUNDERS, vol. 8, no. 6, 1996, pages 376-379, XP004860449 ISSN: 0936-6555
- SMIT ET AL: "An indwelling intrauterine tube to facilitate intracavitary radiotherapy of carcinoma of the cervix" THE BRISTISH JOURNAL OF RADIOLOGY, vol. 62, 1 January 1989 (1989-01-01), pages 68-69, XP009069014
- PHAM ET AL: "Changes in high-dose-rate tandem and ovoid applicator positions during treatment in an unfixed brachytherapy system" RADIOLOGY, vol. 206, no. 2, 1 February 1998 (1998-02-01), pages 525-531, XP002389404
- SMITH M D; TODD J G; SYMONDS R P: "Analgesia for pelvic brachytherapy" BRITISH JOURNAL OF ANAESTHESIA, vol. 88, no. 2, 1 February 2002 (2002-02-01), pages 270-276, XP002389405

## Description

The invention relates to a gynaecological instrument for introducing an irradiation catheter. In particular, the invention relates to a tubular applicator for irradiating gynaecological tumors which is to be placed in the cervix and the bottom of the vagina.

US4434789 shows such an instrument, where irradiation positions are realized by a central catheter which is introduced into the uterus and two laterally pivotable catheters at whose ends the so-called ovoids have been provided and which are pressed against the cervix so as to be pushed apart on the front side of the cervix. The applicator can be connected to a so-called remote afterloading machine which moves a radioactive source to an irradiation position in the tubes via tubes. The applicator usually consists of multiple tubes with two ovoids (ovoid spacers) on the two outer tubes, which ensure that the irradiation doses on the surface remain below the permissible limit. The irradiation is done by bringing a guide source provided at the end of a guide cable in the right position via a guide tube and the catheter and having it emit radiation there for a predetermined period in order to combat the tumor.

The known applicator comprises a central inflatable catheter, which pushes the ovoid structures apart when it is inflated.

Such applicators are often too large to be able to be introduced in an assembled condition and are provided tube by tube and fixed by means of a screw clamp. This is a laborious process and is possibly painful and uncomfortable and often anesthetized, which anesthesia is also carried out differently by different doctors, by which the medical results may be adversely affected.

Therefore, drawbacks are associated with this method because it depends on the skills of the medical staff whether it always yields an optimal result. This constitutes risks for the patient, also because the bladder and the large intestine may thus be exposed to an overmeasure of radiation.

US 5653683 discloses a gynaecological instrument wherein a central rod is embedded in an inflatable balloon for introduction in a vagina. Ovoid structures are described as anchoring balloons that form an external connection to the central rod via a connection plate.

US6312375 discloses a gynaecological instrument comprosing inflatable ovoids Each colpostate/jacket is separately inserted into vagina and positioned, where relative adjustment can be provided. Central catheter is not provided with inflatable jackets.

It is an object of the invention is to provide an instrument where these drawbacks are obviated and where an unambiguous distance from the catheter to the wall of the vagina is provided. At the same time, it is an object of the invention to provide an instrument which can be introduced quickly and with a minimal burden and whose positioning is accurate and reliable.

This object is achieved by an instrument according to the measures of claim 1. In particular, the pneumatic body is mounted around the catheter of the ovoid structure for fixing the catheters with respect to the vaginal wall and for spacing thereof. The catheter can thus be placed as a whole, which is favorable to the ease of placement. The ovoid structure may be part of a ring structure.

In a preferred embodiment, the invention provides a lightweight inflatable disposable plastic applicator which is introduced in one go when empty and is positioned and subsequently inflated, gaining much time and resulting in a smaller burden on the patient. Further, the inflatable body will take over the role of tube gauze and will realize the optimal distance to the organ. After the irradiation, the balloon can deflate and the applicator can be removed in one go.

The pneumatic body provides a fixation and spacing of the catheters so that the radiation sources are introduced by introducing a pneumatic medium, for instance air or liquid. In a preferred embodiment, the body has an inflatable design. Due to the pneumatic character, the body can easily be introduced with a minimal size, after which, by inflating it, it is easily shaped and is fixed in the vagina. After the treatment, the instrument can be taken out. In a next treatment, the instrument can again be introduced easily and in a reproducible manner.

The invention will be explained in more detail with reference to a description of the drawings, in which:
Fig. 1 shows a top plan view and side elevational view, respectively, of a first embodiment of the instrument according to the invention;
Fig. 2 shows a top plan view, side elevational view and front view, respectively, of a second embodiment of the instrument according to the invention in inflated condition;
Fig. 3 shows a top plan view and side elevational view, respectively, of the second embodiment in non-inflated condition;
Fig. 4 shows a top plan view and side elevational view, respectively, of a third embodiment; and
**Fig. 5** shows an embodiment of an annular element which fixes itself to the cervix.

In the Figures, same or corresponding parts are designated by the same reference numerals. Figs. A-C each time show a top plan view, a side elevational view and a front view, respectively.

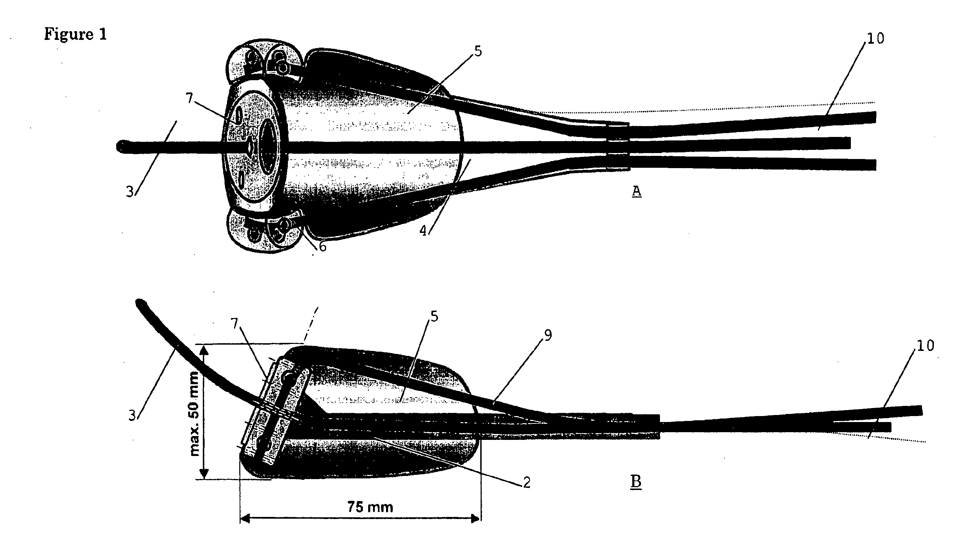

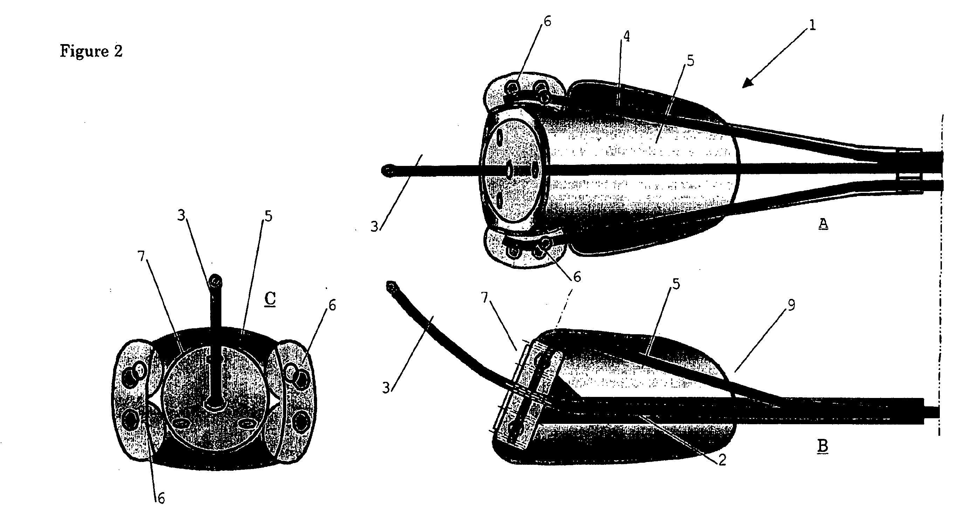

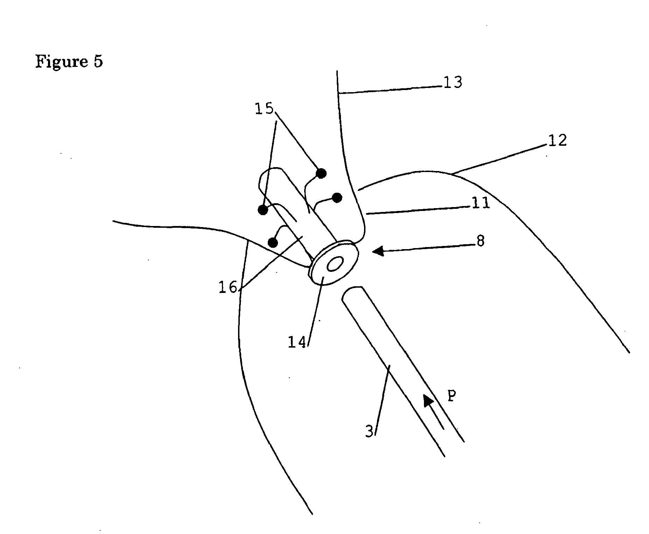

Fig. 1 shows a first embodiment of an applicator 1 according to the invention. What is shown is a tubular central body 2 which serves for introducing a central catheter 3 which is positioned centrally in the cervix for intra-uterine irradiation. Two further tubular guides 4 have been provided on both sides which can be moved away from each other at a distance with respect to a central axis. The tubular guides 4 and the central body 2 are connected with one another by means of an inflatable body 5, which, in inflated condition, can fix the tubes at a fixed distance from each other, as shown in Fig. 1 or Fig. 2. Here, further, the catheters are spaced from the vaginal wall so that the irradiation thereof is limited. Fig. 1 further shows two ovoid structures 6 which are movable from the centre. The ovoid structures 6 have been provided just next to a central stopper 7 which serves to abut the cervix during radiation, in particular, to abut an annular element 8 (see Fig. 5) attached thereon to realize a reproducible fixation of the applicator. A stiffening element 9 can fix the position of the stopper with respect to the longitudinal axis. Around the stopper 7, an inflatable ring may be provided. The annular element 8 may have a design which varies in length depending on the characteristics of the patient and usually comprises sizes of 24, 45 or 65 mm.

The central catheter tube is fixed with respect to the stopper 7 to correspond to this annular element 8. In this position, the catheter to be placed centrally extends farther along a central axis than the ovoid structures 6 which abut the bottom of the vagina during radiation. Here, the central catheter 3 projects into the uterus.

The inflatable body 5 is connected with the stopper 7 and can fix it in the vagina against the cervix by inflating. Here, the inflatable body may comprise at least partly screening material for screening radiation.

The inflatable body can be inflated via a supply tube 10 guided along the catheters.

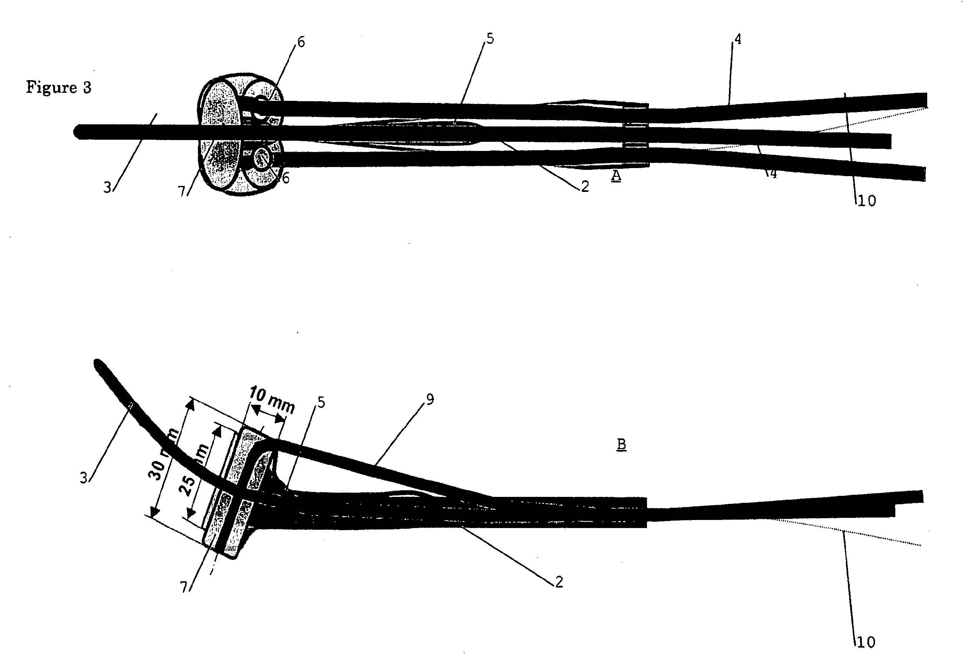

Fig. 3 shows the applicator of Fig. 2 in a non-inflated condition. Here, the cross section of the applicator is minimal and it can be introduced relatively easily.

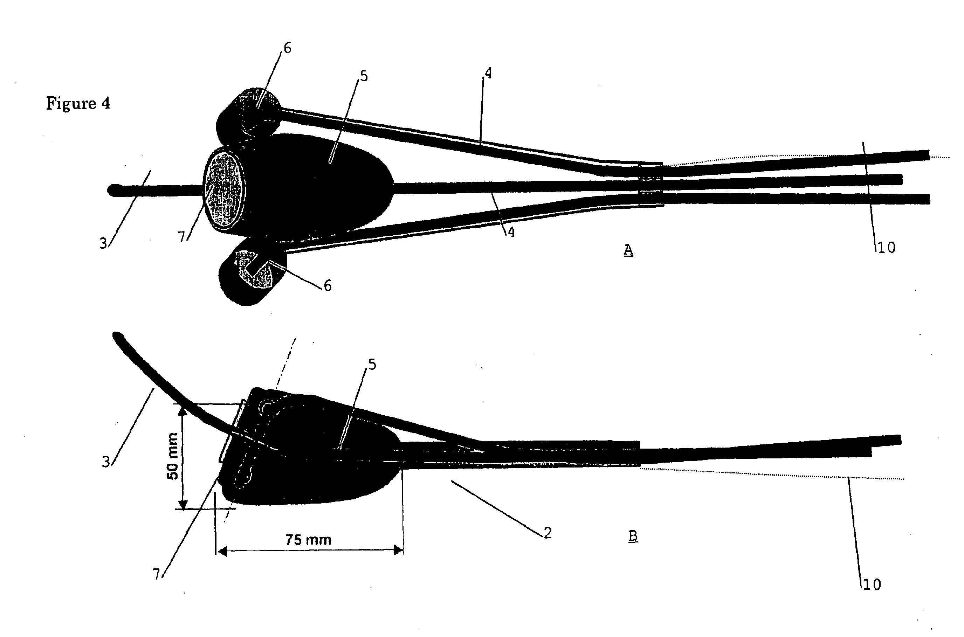

Fig. 4 shows a further embodiment where the ovoid structures 6 also have a pneumatic design. This has the advantage that the applicator can be designed so as to be still smaller in cross section so that it can be introduced still more easily.

Finally, Fig. 5 shows an embodiment of the annular element 8, which comprises a guide tube 16 and a flange 14. The cervix 11, the vaginal bottom wall 12 and the uterus 13 are shown schematically. In the cervix 11, the central guide tube 16 has been provided which has been fixed therein by spring elements 15. The guide tube 16 can stay in place for the period between irradiations and facilitates the introduction of the central catheter 3, schematically shown by means of arrow P.

Although the invention has been explained with reference to the examples shown in the Figures, the invention is not limited thereto but may also comprise variations or modifications without deviating from the intention of the invention. The scope of the invention is determined by the following claims.

## Claims

1. A gynaecological instrument (1) for introducing an irradiation catheter and dimensioned so as to be introduced as a whole, comprising:
- a tubular central body (2) for introduction of a central catheter (3) to be positioned centrally in the cervix;
- a pneumatic or hydraulic body (5) that is inflatable, so that it can fix, in inflated condition, the instrument (1) against the cervix by inflating; and
- a first and second ovoid structures (6) that are movable with respect to a central axis and that are connected to catheter guides (4) and arranged to abut the bottom of the vagina during radiation, wherein the catheter (3) to be positioned centrally extends farther along the central axis than the ovoid structure (6), whereby the pneumatic or hydraulic body (5) is mounted around the catheter guides (4) of the ovoid structures (6); the pneumatic or hydraulic body (5) connects the catheter guide (4) of the ovoid structure to the central body (2) and;
the instrument (1) further comprises a central stopper body (7) for abutting against the cervix, through which the catheter (3) to be positioned centrally is adapted to be fed; and the pneumatic body (5) is connected with the central stopper body (7).

2. A gynaecological instrument according to claim 1, **characterized in that** the ovoid structures (6) are inflatable.

3. A gynaecological instrument according to claim 1, **characterized in that** the length of the central catheter with respect to the central stopper body (7) is adjustable.

4. A gynaecological instrument according to at least one of the preceding claims 1-3, **characterized in that** the pneumatic body (5) has a tubular end through which the catheters (3) are guided and which tubular end is located against the central stopper body (7).

5. A gynaecological instrument according to at least one of the preceding claims, **characterized in that** the pneumatic body (5) comprises at least partly screening material for screening radiation.

6. A gynaecological instrument according to at least one of the preceding claims, **characterized in that** the pneumatic body (5) is put under pressure via a supply tube (10) guided along the catheters.

7. A gynaecological instrument according to at least one of claims 3-6, further comprising an annular element 8 to be fixed on the cervix, in which element a central guide tube (16) is provided for feed-through of the central catheter (3).

8. A gynaecological instrument according to claim 7, wherein the central guide tube (16) comprises at least one spring element (15) which can bend away from the guide tube in order to fix the guide tube in the cervix.

## Patentansprüche

1. Gynäkologisches Instrument (1) zum Einführen eines Bestrahlungskatheters und so dimensioniert, dass es als Ganzes eingeführt werden kann, umfassend:
- einen röhrenförmigen zentralen Körper (2) zum Einführen eines zentralen Katheters (3), der zentral im Gebärmutterhals zu positionieren ist;
- einen pneumatischen oder hydraulischen Körper (5), der aufblasbar ist, sodass er im aufgeblasenen Zustand das Instrument (1) durch Aufblasen gegen den Gebärmutterhals befestigen kann; und
- eine erste und eine zweite eiförmige Struktur (6), die in Bezug auf die Mittelachse beweglich sind und die mit Katheterführungen (4) verbunden sind und angeordnet sind, um während der Bestrahlung gegen die Unterseite der Vagina anzustoßen, wobei der zentral zu positionierende Katheter (3) weiter entlang der Mittelachse verläuft als die eiförmige Struktur (6), wobei der pneumatische oder hydraulische Körper (5) um die Katheterführungen (4) der eiförmigen Strukturen (6) herum befestigt ist; der pneumatische oder hydraulische Körper (5) die Katheterführung (4) der eiförmigen Struktur mit dem zentralen Körper (2) verbindet; und das Instrument (1) ferner einen zentralen Anschlagskörper (7) zum Anstoßen gegen den Gebärmutterhals umfasst, durch den der zentral zu positionierende Katheter (3) zum Einführen geeignet ist; und der pneumatische Körper (5) mit dem zentralen Anschlagskörper (7) verbunden ist.

2. Gynäkologisches Instrument nach Anspruch 1, **dadurch gekennzeichnet, dass** die eiförmigen Strukturen (6) aufblasbar sind.

3. Gynäkologisches Instrument nach Anspruch 1, **dadurch gekennzeichnet, dass** die Länge des zentralen Katheters in Bezug auf den zentralen Anschlagskörper (7) einstellbar ist.

4. Gynäkologisches Instrument nach mindestens einem der vorhergehenden Ansprüche 1-3, **dadurch gekennzeichnet, dass** der pneumatische Körper (5) ein röhrenförmiges Ende aufweist, durch welches die Katheter (3) geführt werden und das gegen den zentralen Anschlagskörper (7) angeordnet ist.

5. Gynäkologisches Instrument nach mindestens einem der vorhergehenden Ansprüche, **dadurch gekennzeichnet, dass** der pneumatische Körper (5) mindestens teilweise Abschirmmaterial zum Abschirmen von Strahlung umfasst.

6. Gynäkologisches Instrument nach mindestens einem der vorhergehenden Ansprüche, **dadurch gekennzeichnet, dass** der pneumatische Körper (5) über eine entlang den Kathetern geführte Zufuhrröhre (10) unter Druck gesetzt wird.

7. Gynäkologisches Instrument nach mindestens einem der Ansprüche 3-6, ferner umfassend ein am Gebärmutterhals zu befestigendes ringförmiges Element (8), in dem eine zentrale Führungsröhre (16) zum Durchführen des zentralen Katheters (3) bereitgestellt ist.

8. Gynäkologisches Instrument nach Anspruch 7, wobei die zentrale Führungsröhre (16) mindestens ein Federelement (15) umfasst, das von der Führungsröhre weg biegen kann, um die Führungsröhre im Gebärmutterhals zu befestigen.

## Revendications

1. Instrument gynécologique (1) pour introduction d'un cathéter d'irradiation et dimensionné de manière à être introduit sous la forme d'un tout, comprenant :
- un corps tubulaire central (2) pour l'introduction d'un cathéter central (3) à positionner de manière centrale dans le col de l'utérus ;
- un corps pneumatique ou hydraulique (5) qui peut être gonflé, de sorte qu'il peut fixer, dans un état gonflé, l'instrument (1) contre le col de l'utérus par gonflage ; et
une première et une seconde structures ovoïdes (6) mobiles par rapport à un axe central et reliées à des guides de cathéter (4) et agencées pour venir en butée contre le fond du vagin pendant un rayonnement, dans lequel le cathéter (3) à positionner de manière centrale s'étend plus loin le long de l'axe central que la structure ovoïde (6), de sorte que le corps pneumatique ou hydraulique (5) est monté autour des guides de cathéter (4) des structures ovoïdes (6), le corps pneumatique ou hydraulique (5) relie le guide de cathéter (4) de la structure ovoïde au corps central (2) et l'instrument (1) comprend en outre un corps de bouchon central (7) pour venir en butée contre le col de l'utérus, à travers lequel le cathéter (3) à positionner de manière centrale est adapté pour être acheminé; et le corps pneumatique (5) est relié au corps de bouchon central (7).

2. Instrument gynécologique selon la revendication 1, **caractérisé en ce que** les structures ovoïdes (6) sont gonflables.

3. Instrument gynécologique selon la revendication 1, **caractérisé en ce que** la longueur du cathéter central par rapport au corps de bouchon central (7) est ajustable.

4. Instrument gynécologique selon au moins l'une des revendications précédentes 1 à 3, **caractérisé en ce que** le corps pneumatique (5) comporte une extrémité tubulaire à travers laquelle les cathéters (3) sont guidés et **en ce que** l'extrémité tubulaire est située contre le corps de bouchon central (7).

5. Instrument gynécologique selon au moins l'une quelconque des revendications précédentes, **caractérisé en ce que** le corps pneumatique (5) comprend au moins partiellement une matière de tamisage, pour tamiser le rayonnement.

6. Instrument gynécologique selon au moins l'une quelconque des revendications précédentes, **caractérisé en ce que** le corps pneumatique (5) est mis sous pression par l'intermédiaire d'un tube d'alimentation (10) guidé le long des cathéters.

7. Instrument gynécologique selon au moins l'une quelconque des revendications 3 à 6, comprenant en outre un élément annulaire (8) à fixer sur le col de l'utérus, élément dans lequel un tube de guidage central (16) est agencé pour la traversée du cathéter central (3).

8. Instrument gynécologique selon la revendication 7, dans lequel le tube de guidage central (16) comprend au moins un élément élastique (15) qui peut se plier loin du tube de guidage pour fixer le tube de guidage dans le col de l'utérus.
